(19) Europäisches Patentamt
European Patent Office
Office européen des brevets

(11) Veröffentlichungsnummer: **0 122 355 A2**

(12) # EUROPÄISCHE PATENTANMELDUNG

(21) Anmeldenummer: 83810335.6

(22) Anmeldetag: 25.07.83

(51) Int. Cl.³: C 07 D 241/24

(30) Priorität: 21.03.83 CH 1514/83

(43) Veröffentlichungstag der Anmeldung: 24.10.84 Patentblatt 84/43

(84) Benannte Vertragsstaaten: CH DE FR GB IT LI NL SE

(71) Anmelder: Servipharm Ltd.
Postfach
CH-4002 Basel(CH)

(72) Erfinder: Zergényi, Janos, Dr.
Jurastrasse 31
CH-4411 Seltisberg(CH)

(72) Erfinder: Räz, Bernhard
Sommergasse 39
CH-4056 Basel(CH)

(74) Vertreter: Meyer, Hans Rudolf et al,
Patentabteilung der CIBA-GEIGY AG Postfach
CH-4002 Basel(CH)

(54) **Verfahren zur Herstellung von Pyrazinamid.**

(57) Die Erfindung betrifft ein neuerartiges Verfahren zur Herstellung von Pyrazinamid, demzufolge man Chlorpyrazin in einem N-alkylierten aliphatischen Amid oder Lactam mit einem Alkalimetallfluorid zu Fluorpyrazin umsetzt, dieses durch Umsetzung mit einem Alkalimetalloder Erdalkalimetallcyanid in Cyanopyrazin überführt und dieses durch Behandeln mit annähernd konzentrierter Schwefelsäure und anschliessend mit Wasser in Pyrazinamid umwandelt.

EP 0 122 355 A2

Croydon Printing Company Ltd.

SERVIPHARM Ltd.
Basel (Schweiz)

4-14359

Verfahren zur Herstellung eines Amids

Die Erfindung betrifft ein neuartiges Verfahren zur Herstellung von Pyrazinamid der Formel

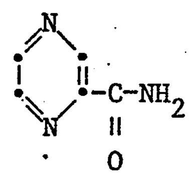

(I).

Pyrazinamid ist ein wichtiger antibakterieller, insbesondere tuberkulostatischer Arzneimittelwirkstoff, der üblicherweise ausgehend von Chinoxalin durch Oxidation zur Pyrazin-2,3-dicarbonsäure, Ueberführung derselben in das Anhydrid, dessen Alkoholyse zum Monomethylester und Decarboxylierung desselben bzw. Decarboxylierung und Veresterung zum Pyrazincarbonsäuremethylester und Ammonolyse desselben hergestellt wird.

Dieses Verfahren hat indes gewichtige Nachteile. Insbesondere kommen als Oxidationsmittel für die Oxidation von Chinoxalin zu Pyrazin-2,3-dicarbonsäure praktisch lediglich Mangan-VII-verbindungen, vor allem Alkalimetallpermanganate, in Betracht, da andere Oxidationsmittel nicht zum Erfolg führen oder nicht in technischem Massstab angewendet werden können. So wird bei der Oxidation von Chinoxalin mit Wasserstoffperoxid lediglich das Chinoxalin-1,4-dioxid, mit Salpetersäure unter Nitrierung vor allem ein Gemisch verschiedener Nitro-1,2,3,4-tetrahydro-chinoxalin-2,3-dione erhalten. Die Ozonbehandlung von Chinoxalin soll zwar in befriedigender Ausbeute Pyrazin-2,3-dicarbonsäure ergeben, konnte aber bislang nicht reproduziert werden; das Verfahren würde zudem erhebliche apparative sowie

sicherheitstechnische Aufwendungen erfordern und wäre deshalb nicht wirtschaftlich.

Bei der Oxidation von Chinoxalin mit Kaliumpermanganat entstehen aber als Nebenprodukte die äquimolekulare Menge an Kaliumoxalat sowie die 6-fache Menge an Mangandioxid. Letzteres muss abfiltriert und entsorgt bzw. zur andersweitigen Verwendung gereinigt werden. Im Filtrat liegt die Pyrazin-2,3-dicarbonsäure als Kaliumsalz vor; sie muss als Bariumsalz ausgefällt und aus diesem mit Schwefelsäure freigesetzt werden.

Es hat deshalb an Versuchen nicht gefehlt, die aufgezeigten Schwierigkeiten durch einen grundsätzlich andersartigen Syntheseweg zu umgehen. So schlägt die japanische Patentanmeldung Nr. 82/11.917 vor, von Cyanopyrazin auszugehen und dieses durch 1,5-stündiges Erhitzen mit 28%-iger wässriger Ammoniaklösung auf 80 - 90°C in Pyrazinamid zu überführen. Derzeit steht jedoch kein im technischen Massstab wirtschaftliches Verfahren für die Herstellung des Ausgangsproduktes zur Verfügung. Das Aminooxidationsverfahren der japanischen Patentanmeldung Nr. 80/145.672 beispielsweise geht von dem herstellungsaufwendigen Methylpyrazin aus und erfordert zudem einen speziellen Mehrkomponentenkatalysator. Die Ueberführung von Jod- bzw. Brompyrazin in Cyanopyrazin gemäss J. Heterocyclic Chem. 2, 209 (1965) erfordert Kupfer-I-cyanid, das teuer und dessen Verwendung ökologisch bedenklich ist. Zudem ist die ausgehend von Brompyrazin erzielte Ausbeute unbefriedigend. Die Umsetzung von Jodpyrazin mit Kupfer-I-cyanid verläuft zwar glatter; dafür verläuft aber die Herstellung von Jodpyrazin aus Chlorpyrazin gemäss J. Org.Chem. 26, 1907 (1961) mit unbefriedigender Ausbeute. Im Prinzip wäre zwar denkbar, dass auch Chlorpyrazin mit Kuper-I-cyanid in Cyanopyrazin überführt werden könnte. Jedoch ist in der Literatur nichts darüber bekannt. Da die Ausbeute der Reaktion bereits von Jod- zu Brompyrazin stark abnimmt, muss auch bezweifelt werden,

dass diese Reaktionsweise auf Chlorpyrazin übertragbar sei.

Es besteht also nach wie vor Bedarf nach einem fortschrittlichen Herstellungsverfahren für Pyrazinamid, welches die geschilderten Nachteile der bestehenden umgeht, von leicht zugänglichen Ausgangsstoffen ausgeht, einfach und ohne Bildung ökologisch bedenklicher Abfallprodukte durchzuführen und im technischen Massstab wirtschaftlich ist.

Diese Aufgabe wird erfindungsgemäss dadurch gelöst, dass man Chlorpyrazin in einem N-alkylierten aliphatischen Amid bzw. Lactam mit einem Alkalimetallfluorid zu Fluorpyrazin umsetzt, dieses durch Umsetzung mit einem Alkalimetall- oder Erdalkalimetallcyanid in Cyanopyrazin überführt und dieses durch Behandeln mit annähernd konzentrierter Schwefelsäure und anschliessend mit Wasser in Pyrazinamid umwandelt.

Der Erfindung liegen die überraschenden Feststellungen zugrunde, dass die Umsetzung von Chlorpyrazin mit einem Alkalimetallfluorid in einem N-alkylierten aliphatischen Amid bzw. Lactam mit besonders hoher Ausbeute verläuft, dass Fluorpyrazin mit einem Alkalimetall- bzw. Erdalkalimetallcyanid im Gegensatz zu Chlorpyrazin glatt zu Cyanopyrazin umgesetzt werden kann,und dass die Hydrolyse desselben in annähernd konzentrierter Schwefelsäure mit sehr hoher Ausbeute besonders reines Pyrazinamid liefert. Das erfindungsgemässe Verfahren hat den Vorteil, dass durch die vorgeschlagenen Massnahmen bei der Ueberführung sowohl von Chlorpyrazin in Fluorpyrazin als auch von Cyanopyrazin in Pyrazinamid höhere Ausbeuten und/oder reinere Produkte erzielt werden als bei den entsprechenden bekannten Verfahrensvarianten,und dass die Umsetzung von Fluorpyrazin zu Cyanopyrazin in überraschender Weise mit Alkalimetall bzw. Erdalkalimetallcyaniden bewirkt werden kann, wobei als anorganische Nebenprodukte lediglich ökologisch unbedenkliche, weil leicht zu entsorgende Alkalimetall- bzw. Erdalkalimetallhalogenide anfallen. Die Erfindung betrifft dementsprechend sowohl das Gesamtverfahren als auch jeden der individuellen Teilschritte.

N-alkylierte aliphatische Amide bzw. Lactame, welche als Lösungs- oder Verdünnungsmittel für die Umsetzung von Chlor- zu Fluorpyrazin geeignet sind, sind beispielsweise N,N-Diniederalkyl-niederalkansäureamide bzw. N-Niederalkyl-niederalkansäurelactame, z.B. N,N-Dimethylacetamid, N,N-Diäthylformamid oder insbesondere N-Methylpyrrolidon. Alkalimetallfluoride sind beispielsweise Natrium- oder insbesondere Kaliumfluorid. Die Umsetzung erfolgt vorzugsweise unter Erwärmen, beispielsweise auf etwa 140 - 200°C, insbesondere auf etwa 170 - 190°C, vorteilhaft unter Abdestillieren des gebildeten Fluorpyrazins. Das Lösungsmittel kann praktisch quantitativ regeneriert werden, so dass als Abfallprodukte lediglich Alkalichlorid,sowie höchstens geringe Mengen Alkalifluorid anfallen.

In einer bevorzugten Ausführungsform dieses Verfahrensschrittes erhitzt man ein annähernd äquimolekulares Gemisch von Chlorpyrazin und Kaliumfluorid in N-Methylpyrrolidon auf etwa 180°C, bis kein Fluorpyrazin mehr übergeht (etwa 24 Stunden).

Für die Umsetzung mit Fluorpyrazin geeignete Alkalimetall- bzw. Erdalkalimetallcyanide sind beispielsweise Kalium- oder insbesondere Natriumcyanid, ferner Calciumcyanid. Da Cyanopyrazin baseempfindlich ist, wird das Fluorpyrazin vorteilhaft im Ueberschuss, beispielsweise in etwa 1,2- bis etwa 1,7-facher, insbesondere in etwa 1,5-facher molarer Menge verwendet. Der Ueberschuss kann recycliert werden. Die Umsetzung wird vorteilhaft in einem aprotischen, polaren Lösungs- oder Verdünnungsmittel unter Erwärmen durchgeführt. Geeignete Lösungs- oder Verdünnungsmittel sind beispielsweise N-niederalkylierte aliphatische Amide bzw. Lactame, z.B.

N,N-Dimethylformamid, ferner N,N-Dimethylacetamid oder N-Methylpyrrolidon, oder aliphatische bzw. cycloaliphatische Sulfoxide oder Sulfone, z.B. Dimethylsulfoxid. Die Reaktion verläuft am besten im Temperaturbereich von etwa 60 bis etwa 140°C, abhängig von der Art des verwendeten Lösungs- oder Verdünnungsmittels. Bei Verwendung von Dimethylformamid hat sich eine Reaktionstemperatur von etwa 100 bis 140°C, z.B. von etwa 120°C, bei Verwendung von Dimethylsulfoxid eine Reaktionstemperatur von etwa 60 bis 100°C, z.B. von etwa 80°C, als besonders günstig erwiesen.

In einer bevorzugten Ausführungsform erwärmt man eine Lösung von Natriumcyanid und der etwa 1,5-fachen Menge an Fluorpyrazin in N,N-Dimethylformamid etwa 40 Minuten auf etwa 120°C, destilliert dann unter vermindertem Druck das überschüssige Fluorpyrazin zusammen mit dem Lösungsmittel ab und unterwirft den Rückstand der fraktionierten Destillation unter vermindertem Druck, um das gebildete Cyanopyrazin abzutrennen. Der Vorlauf kann recycliert, d.h. im nächsten Ansatz eingesetzt, werden; der Rückstand besteht im wesentlichen aus Natriumfluorid neben geringen Mengen an Natriumcyanid.

Im Zusammenhang mit der Umwandlung von Cyanopyrazin in Pyrazinamid ist unter annähernd konzentrierter Schwefelsäure etwa 90 %-ige bis etwa 99,6 %-ige, vorzugsweise etwa 96 %-ige Schwefelsäure, zu verstehen. Die Umsetzung erfolgt vorzugsweise, indem man das Cyanopyrazin bei etwa 30 bis 40°C in der Schwefelsäure löst, die Lösung in Wasser einrührt, neutralisiert und das ausgefallene Pyrazinamid abfiltriert. Die Neutralisierung kann mit einer beliebigen Base, beispielsweise mit Ammoniumhydroxid, erfolgen.

Die Erfindung wird durch die nachfolgenden Beispiele näher erläutert. Drucke sind in mbar, Temperaturen in Celsiusgraden angegeben.

Beispiel 1: Herstellung von Fluorpyrazin aus Chlorpyrazin

200 g Kaliumfluorid-dihydrat werden unter Rühren in 500 ml N-Methylpyrrolidon eingetragen und solange am Wasserabscheider erhitzt, bis azeotrop kein Wasser mehr abdestilliert. Dann lässt man innerhalb von 30 Minuten 200 g Chlorpyrazin hinzutropfen, hält die Reaktionslösung noch 1 Stunde am Rückfluss und destilliert den bei 120-130° siedenden Teil ab, wobei 159,3 g Rohprodukt übergehen. Dieses wird über eine Kolonne feindestilliert, wobei bei 108° 143 g (83,8 % d.Th.) reines Fluorpyrazin übergeht.

Beispiel 2: Herstellung von Cyanopyrazin aus Fluorpyrazin

Variante a): Ein Gemisch von 98,1 g Fluorpyrazin, 32,7 g Natriumcyanid und 385 g N,N-Dimethylformamid werden in einem 1L-Rundkolben 40 Minuten auf 120° erwärmt. Man lässt auf Raumtemperatur abkühlen, versetzt mit 50 g Paraffinöl und destilliert fraktioniert unter vermindertem Druck. Dabei werden 3 Fraktionen mit folgenden Charakteristiken aufgefangen:

| Fraktion | Siedebereich | Druck | Menge | Gehalt* |
|---|---|---|---|---|
| 1 | 54-64 | 20 | 375,3 g | 28,9 g Fluorpyrazin<br>3,05 g Cyanopyrazin |
| 2 | 64-74 | 20-5 | 73,3 g | 21,4 g Cyanopyrazin |
| 3 | 74-122 | 5 | 29,4 g | 26,4 g Cyanopyrazin |

* gaschromatographisch ermittelt

Man erhält somit 50,85 g (76 % d.Th. bezogen auf umgesetztes Fluorpyrazin) Cyanopyrazin neben 28,9 g nicht umgesetztem Fluorpyrazin und 398 g rohem N,N-Dimethylformamid.

Variante b): Ein Gemisch von 98,1 g Fluorpyrazin, 32,7 g Natriumcyanid und 385 g Dimethylsulfoxid werden in einem mit Magnetrührer und Destillationsaufsatz versehenen 1 L-Rundkolben unter Rühren 40 Minuten auf 80° erwärmt. Dann lässt man etwas abkühlen und destilliert fraktioniert unter vermindertem Druck. Dabei werden 4 Fraktionen mit folgenden Charakteristiken erhalten:

| Fraktion | Siedebereich | Druck | Menge | Gehalt * |
|---|---|---|---|---|
| 1 | 43-55° | 100-2 | 18,18 g | 14,77 g Fluorpyrazin |
| | | | | 0,19 g Cyanopyrazin |
| 2 | 55-32 | 2-0,1 | 207,48 g | 19,73 g Fluorpyrazin |
| | | | | 14,73 g Cyanopyrazin |
| 3 | 32-34 | 0,1 | 241,09 g | 38,65 g Cyanopyrazin |
| 4 | 34-33 | 0,1 | 4,07 g | 1.52 g Cyanopyrazin |

* gaschromatographisch ermittelt

Man erhält also 55,09 g (83,6 % d.Th. bezüglich umgesetzten Fluorpyrazins) Cyanopyrazin neben 34,5 g nicht umgesetztem Fluorpyrazin und 382 g Dimethylsulfoxid.

Vergleichsbeispiel 2: Umsetzung von Chlorpyrazin mit Alkalicyanid

Ein Gemisch von 11,45 g Chlorpyrazin, 4,34 g Kaliumcyanid und 35 ml Dimethylsulfoxid wurde in einem Rundkolben 60 Minuten auf 80° erwärmt. In Abständen von jeweils 20 Minuten wurde eine Probe entnommen und gaschromatisch auf Cyanopyrazin untersucht. Dabei konnten nur geringe Spuren (entsprechend einer Ausbeute von

weniger als 1 % d.Th.) Cyanopyrazin nachgewiesen werden.

Beispiel 3: Herstellung von Pyrazinamid aus Cyanopyrazin

10 g Cyanopyrazin werden unter Rühren und Kühlen auf 20° in 20 ml 96 %-ige Schwefelsäure eingetragen. Man lässt 4 Stunden bei 25° rühren, giesst auf 100 g Eis und neutralisiert mit 80 ml konzentrierter Ammoniumhydroxyidlösung, worauf sich ein kristalliner Niederschlag bildet. Man lässt 2 Stunden bei 0° nachrühren, saugt ab, wäscht mit Wasser und trocknet unter vermindertem Druck bis zur Gewichtskonstanz. Man erhält 8,3 g (80,5 % d.Th) Pyrazinamid. Aus der Mutterlauge können weitere 1,7 g gewonnen werden, so dass die erzielte Gesamtausbeute 97 % d.Th. beträgt.

Patentansprüche

1. Verfahren zur Herstellung von Pyrazinamid der Formel

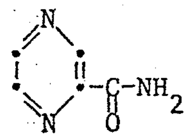

(I)

dadurch gekennzeichnet, dass man Chlorpyrazin in einem N-alkylierten aliphatischen Amid oder Lactam mit einem Alkalimetallfluorid zu Fluorpyrazin umsetzt, dieses durch Umsetzung mit einem Alkalimetall- oder Erdalkalimetallcyanid in Cyanopyrazin überführt und dieses durch Behandeln mit annähernd konzentrierter Schwefelsäure und anschliessend mit Wasser in Pyrazinamid umwandelt.

2. Verfahren zur Herstellung von Fluorpyrazin durch Umsetzung von Chlorpyrazin mit einem Alkalimetallfluorid, dadurch gekennzeichnet, dass man diese in einem N-alkylierten aliphatischen Amid oder Lactam durchführt.

3. Verfahren zur Herstellung von Cyanopyrazin, dadurch gekennzeichnet, dass man Fluorpyrazin im Ueberschuss mit einem Alkalimetall- oder Erdalkalimetallcyanid umsetzt.

4. Verfahren zur Herstellung von Pyrazinamid aus Cyanopyrazin, dadurch gekennzeichnet, dass man Cyanopyrazin mit annähernd konzentrierter Schwefelsäure und anschliessend mit Wasser behandelt.

5. Verfahren nach Anspruch 1 oder 2, dadurch gekennzeichnet, dass man als N-alkyliertes aliphatisches Amid bzw. Lactam ein N,N-Diniederalkyl-niederalkansäureamid oder ein N-Niederalkyl-niederalkansäurelactam verwendet.

6. Verfahren nach Anspruch 1 oder 2, dadurch gekennzeichnet, dass man als N-alkyliertes aliphatisches Lactam N-Methylpyrrolidon verwendet.

7. Verfahren nach einem der Ansprüche 1, 2, 5 und 6, dadurch gekennzeichnet, dass man Chlorpyrazin in N-Methylpyrrolidon bei etwa 140 bis etwa 200°C, insbesondere etwa 170 bis etwa 190°C, mit Kaliumfluorid umsetzt.

8. Verfahren nach Anspruch 7, dadurch gekennzeichnet, dass man das gebildete Fluorpyrazin durch Destillation aus dem Reaktionsgemisch abtrennt.

9. Verfahren nach Anspruch 1 oder 3, dadurch gekennzeichnet, dass man als Alkalimetall- bzw. Erdalkalimetallcyanid Natriumcyanid, Kaliumcyanid oder Calciumcyanid verwendet.

10. Verfahren nach Anspruch 1, 3 oder 9, dadurch gekennzeichnet, dass man die Umsetzung in einem aprotischen, polaren Lösungs- oder Verdünnungsmittel vornimmt.

11. Verfahren nach Anspruch 10, dadurch gekennzeichnet, dass man als aprotisches, polares Lösungsmittel ein N-niederalkyliertes aliphatisches Amid bzw. Lactam oder ein aliphatisches oder cycloaliphatisches Sulfoxid oder Sulfon verwendet.

12. Verfahren nach Anspruch 10, dadurch gekennzeichnet, dass man als aprotisches, polares Lösungs- oder Verdünnungsmittel N,N-Dimethylformamid, N,N-Dimethylacetamid, N-Methylpyrrolidon oder Dimethylsulfoxid verwendet.

13. Verfahren nach einem der Ansprüche 1 und 9 bis 12 dadurch gekennzeichnet, dass man im Temperaturbereich von etwa 60 bis etwa 140°C arbeitet.

14. Verfahren nach einem der Ansprüche 1 und 9 bis 13 dadurch gekennzeichnet, dass man das Fluorpyrazin in etwa 1,3- bis etwa 1,7-fachem Ueberschuss einsetzt.

15. Verfahren nach einem der Ansprüche 1 und 9 - 14 dadurch gekennzeichnet, dass man Fluorpyrazin im etwa 1,5-fachem Ueberschuss bei etwa 100 bis etwa 140°C in N,N-Dimethylformamid bzw. bei etwa 60 bis etwa 100° in Dimethylsulfoxid mit Natriumcyanid umsetzt.

16. Verfahren nach Anspruch 15, dadurch gekennzeichnet, dass man das nicht umgesetzte Fluorpyrazin zusammen mit dem N,N-Dimethylformamid abdestilliert.

17. Verfahren nach Anspruch 1 oder 4, dadurch gekennzeichnet, dass man als annähernd konzentrierte Schwefelsäure etwa 96 %-ige Schwefelsäure verwendet.

18. Das Verfahren der Beispiele 1 bis 3.

19. Die nach dem Verfahren der Ansprüche 1 - 18 erhaltenen Verbindungen bzw. Fluorpyrazin, Cyanopyrazin und/oder Pyrazinamid, sofern erhalten nach dem Verfahren der Ansprüche 1 - 18.